Europäisches Patentamt

European Patent Office

Office européen des brevets

(11) Publication number: **0 423 240 B1**

## EUROPEAN PATENT SPECIFICATION

(45) Date of publication of patent specification: **08.11.95**

(51) Int. Cl.⁶: **A61K 31/735**

(21) Application number: **89909095.5**

(22) Date of filing: **06.07.89**

(86) International application number:
**PCT/US89/02944**

(87) International publication number:
**WO 90/00596 (25.01.90 90/03)**

(54) **MODULATION OF VIRUS-HOST CELL INTERACTIONS USING CYCLIC OLIGOSACCHARIDES.**

(30) Priority: **07.07.88 US 216176**
**28.06.89 US 373005**

(43) Date of publication of application:
**24.04.91 Bulletin 91/17**

(45) Publication of the grant of the patent:
**08.11.95 Bulletin 95/45**

(84) Designated Contracting States:
**AT BE CH DE FR GB IT LI LU NL SE**

(56) References cited:
**US-A- 4 783 446**

**Antimicrobial Agents and Chemotherapy, Volume 31, issued October 1987, NAKASHIMA et al., "Purification and Characterization of an Avian Immuno-Deficiency Virus Reverse Transcriptase Inhibitor, Sulfated Polysacharides Extracted from Sea Algae, pages 1524-1528**

(73) Proprietor: **THE TRUSTEES OF THE UNIVERSITY OF PENNSYLVANIA**
**Mellon Bank Building,**
**133 South 36th Street,**
**Suite 419**
**Philadelphia, Pennsylvani 19104 (US)**

(72) Inventor: **ROTH, Steven, A.**
**1105 Rose Glen Road**
**Gladwyne, PA 19035 (US)**
Inventor: **WEISZ, Paul, B.**
**3 Delaware Rim Drive**
**Yardley, PA 19067 (US)**
Inventor: **FARNBACH, George**
**132 Rutgers Avenue**
**Swarthmore, PA 19081 (US)**
Inventor: **WEINER, David B.**
**23 Henley Road**
**Overbrook Hill, PA 19151 (US)**
Inventor: **GREENE, Mark, I.**
**300 Righters Mill Road**
**Penn Valley, PA (US)**

EP 0 423 240 B1

Rank Xerox (UK) Business Services
(3.10/3.09/3.3.3)

Science, Volume 240, issued 29 April 1988, MITSUYA et al., "Dextran Sulfate Suppression of Viruses in the HIV Family: Inhibition of Virion Binding to CD4+ Cells, pages 646-649

Inventor: **WILLIAMS, William V.**
**25 Sycamore Road**
**Havertown, PA 19083 (US)**

(74) Representative: **Hirsch, Marc-Roger**
**Cabinet Hirsch**
**34 rue de Bassano**
**F-75008 Paris (FR)**

**Description**

1. FIELD OF THE INVENTION

The present invention relates to the field of treatments for infection by viruses. More particularly, this invention relates to the field of biologically active agents which interfere with or inhibit virus-host cell interactions by binding to receptor site(s) on the host cells or on viruses or both.

2. BACKGROUND OF THE INVENTION

2.1. HIV

Acquired Immune Deficiency Syndrome (AIDS) is one of the most feared diseases in the world today. Infection with the human immunodeficiency virus (HIV-1), believed to be the cause of AIDS is almost always fatal. Symptoms of the disease can take years to develop, thus facilitating the spread of this fatal disease by persons unknowingly harboring the virus. Treatments for AIDS are limited and have been unsuccessful in controlling the disease.

It is generally recognized that an envelope glycoprotein of HIV-1 plays a key role in the process of infection of human tissue cells by the virus. The envelope glycoprotein consists of two main glycoprotein portions-gp120 and gp41. The gp120 is believed to be the outermost part of the complex made up of these two glycoproteins. It is believed that the binding of the HIV-1 particle to human cells, a key event in the infection of cells with the virus, requires binding between gp120 and a receptor protein complex known as CD4 on certain human cells.

CD4 expression on human cells has been demonstrated to increase susceptibility to HIV-1 binding, penetration and infection. CD4, a 55,000 dalton cell surface glycoprotein is thought to represent a cell surface receptor to which HIV-1 envelope glycoprotein (gp120) binds. This interaction likely facilitates events necessary for viral fusion with the plasma membrane and subsequent entry into the cell.

It is believed that there are at least three separate binding sites on the gp120 molecule which combine to form a high affinity binding site for the CD4 receptor. This affinity for the CD4 receptor is extremely strong and this binding affinity may displace or prevent binding of antibodies produced by the host in standard immunological defense. For example, most AIDS patients and most ARC (AIDS Related Complex) patients have high levels of antibody to gp120, however the gp120-CD4 interaction may displace bound antibody so that vaccines directed against gp120 can be expected to be ineffective since the antibodies they elicit from the host will be relatively ineffective. One commercially available compound which is useful as an anti-infective agent is a form of CD4. The compound is believed to work because it binds the gp120 as tightly as the natural CD4. In this way, if given in high enough concentrations, the free (administered) CD4 will bind all of the viral gp120 and prevent its binding CD4 on host cells.

The HIV-1 virus has been shown to infect preferentially cells expressing the CD4 surface glycoprotein. This tropism is believed to result from interactions between the virus envelope gp120 and a CD4 high affinity binding site glycoprotein which permits viral absorption. Following the initial attachment of virus to the cell surface CD4 molecule, other regions of the virus envelope are believed to be important in initiating fusion between the viral and cellular membranes. This interaction is thought to precede viral entry, uncoating, and replication.

Although there is much evidence to support the foregoing proposed sequence of events, several studies have demonstrated that CD4 may be necessary, but not sufficient, to mediate entry of HIV-1 into cells. These experiments have used primary cells expressing CD4 and human cell lines transfected with CD4; these cells bind and internalize HIV-1 virus. Additionally, monocytes and certain rare B cell lines which are infectable by HIV-1 express mRNA potentially encoding CD4 proteins. Finally, antibodies to specific CD4 epitopes inhibit viral attachment and syncytia formation as well as viral infection.

Most murine cells expressing the human CD4 glycoprotein bind HIV-1 but do not readily form syncytia and are not readily infected. Additionally, it has been reported that antibodies that disrupt viral-CD4 interactions do not block viral infection of certain human neuronal cell lines. These latter observations suggest that other factors and or other molecules present on human cells in addition to CD4 may be operative in HIV-1 penetration.

Cell surface receptors are known to occur on many types of cells. These receptors are believed to be important in a wide variety of cellular functions such as cell-cell recognition processes, growth regulation, metabolic and hormonal regulation and structural aggregation of cells. Some of these cell surface receptors, such as the CD4 receptor, are considered to be glycoproteins. Glycoproteins are molecules comprising a protein moiety linked with a Polysaccharide moiety. Typically, the protein moiety constitutes the greater part of the molecule. The ligands of some receptors, such as gp120, the envelope glycoprotein of HIV-1 which binds to the receptor CD4, are also glycoproteins. It is believed that the polysaccharide portion of the glycoprotein molecule plays a role in the interaction between the receptor and its ligand and is important for imparting specificity to the receptor-ligand binding in at least some instances.

2.2. POLYSACCHARIDES AS THERAPEUTIC AGENTS

Polysaccharides are biologically important polymers made up of linked single sugar molecules. These substances are ubiquitous in biological organisms. Starch is a commonly known polysaccharide as are many naturally-occurring compositions. Small segments of polysaccharide chains are known to be important in the physical and chemical properties of many proteins-particularly in eukaryotic organisms. Various polysaccharides and their derivatives are known to be important in surface phenomena in biological systems. Heparin, for example is a mixture of polysulfated polysaccharides with molecular weights ranging from about 5,000 to 40,000; it is commercially prepared from biological material such as lung tissue. The substances contained in heparin are important biological surfactants and also prevent clot formation in blood. Heparin and heparin-like substances are also known to prevent the adhesion of potentially infectious bacteria to surfaces in the urinary bladder, urethra, and ureters.

Although heparin has long been used in medicine to treat blood clots, heparin has recently received attention by researchers for other uses. Science Focus, New York Academy of Sciences, 2(3): 1, 1988 reports that heparin can inhibit HIV, the viral agent that causes AIDS, in selected cells in one stage of the virus's reproductive cycle. However, heparin is a well-known, strong anticoagulant and its continued use in mammals can lead to bleeding disorders. Further, commercial heparin is a heterogeneous mixture of substances derived from natural sources.

Mitsuya et al., Science, 240: 646-649, (1988), have demonstrated that the use of high molecular weight (approximately 8,000 M.W.) poly-sulfated, linear polydextrans can inhibit the infection by HIV-1 of certain human white blood cells. Baba et al., Proc. Natl. Acad. Sci. U.S.A. 85. 6132-6136 have demonstrated that dextran sulfate (5000 m.w.) and heparin can inhibit the infection by HIV-1 of certain white blood cells. U.S. Patent N° 4,783,446, 1988, discloses the use of carrageenans or mixtures of carrageenans to treat AIDS and other retrovirus infections. The carrageenans will be in the range of 5,000-500,000 M.W. with most ranging from 100,000 to 500,000 M.W. However, it is not clear whether this phenomenon is a specific or non-specific effect of this compound, since such large molecular weight substances of this type may have non-specific membrane effects on the lipid properties of the cell surface and the viral envelope surface. Further, like heparin, the high molecular weight compound is strongly anticoagulant.

Treatment of individuals infected with HIV-1 has been complicated by the binding capacity of the virus to mammalian cells and the extreme toxicity of infection with the virus. There is a potential for inadvertently infection healthy individuals with only partially inactivated whole HIV-1 or components of the virus as part of a vaccine. Efforts to control the virus through drugs has not succeeded to date. Alternative means of treating individuals infected with HIV-1, as well as alternate means of preventing or inhibiting infection of cells with HIV-1 are needed which are not toxic to the individual infected with HIV-1 and are safe for individuals not infected with the virus.

3. SUMMARY OF THE INVENTION

The present invention provides novel uses for inhibiting infection of cells by a virus using a carbohydrate as a blocking agent. The invention is also directed to compositions and methods for inhibiting the cell to cell transmission of the virus using a carbohydrate as a blocking agent. The carbohydrate blocking agent is capable of interacting with the host cells and interferes with binding of the virus to the cells. Preferably, the carbohydrate blocking agent is a very water soluble derivative of a cyclic oligosaccharide having up to about twelve sugar monomers. The cyclic oligosaccharide derivative may contain ionic and/or non-ionic substitutes. As used in the present application, the terme "very water soluble" is intended to encompass a solubility, measured at 0°C, of at least about 20 g/100 ml in distilled water, more preferably greater than 30 gm/100 ml. Preferably, the cyclic oligosaccharide has about six to eight sugar monomers.

This invention also provides the use of a composition for inhibiting syncytia formation between cells comprising providing a carbohydrate blocking agent which is capable of interacting with said cells or virus, said carbohydrate blocking agent comprising a cyclic oligosaccharide derivative having up to about twelve sugars wherein said derivative is characterized by a solubility of at least 20 g/100 ml. The agent is administered to said cells under conditions selected to effect interaction of the cyclic oligosaccharide

EP 0 423 240 B1

derivative with said cells thereby inhibiting formation of syncytia. In another embodiment, the cyclic oligosaccharide derivatives of the present invention may inhibit syncytia formation by administering the carbohydrate blocking agent under conditions where the virus is blocked from binding to the cell.

This invention further provides the use of a composition for inhibiting virus infection in mammals including humans, comprising (1) a very water-soluble derivative of a cyclic oligosaccharide in combination with (2) at least one antiviral agent, said derivative being characterized by a solubility in distilled water of at least about 20 grams per 100 milliliters of water at 0°C. The compositions may also be used to inhibit cell to cell transmission of the virus. The compositions in another embodiment may be used to inhibit syncytia formation in infected cells.

The invention additionally provides the use of a composition for inhibiting retrovirus infection in mammals including humans comprising (1) a very water-soluble derivative of a cyclic oligosaccharide in combination with (2) at least one cell growth modulating steroid or non-steroidal organic compound, wherein the steroid compound is devoid of glucocorticoid activity, and said derivative is characterized by a solubility in distilled water of at least 20 grams per 100 milliliters of water at 0°C. In one embodiment, these compositions may be used to treat Kaposi's sarcoma, often manifested in AIDS patients. The compositions may also be used to prevent cell to cell transmission of the virus.

Further provided is the use of a composition for inhibiting retrovirus infection in mammals including humans, comprising (1) a very water-soluble derivative of a cyclic oligosaccharide in combination with (2) at least one antiviral agent and (3) at least one growth modulating steroid or non-steroidal organic compound, wherein the steroid compound is devoid of glucocorticoid activity, and said derivative is characterized by a solubility in distilled water of at least 20 grams per 100 milliliters of water of 0°C. In one embodiment, these compositions may be used to treat Kaposi's sarcoma, often manifested in AIDS patients.

## 4. BRIEF DESCRIPTION OF THE FIGURES

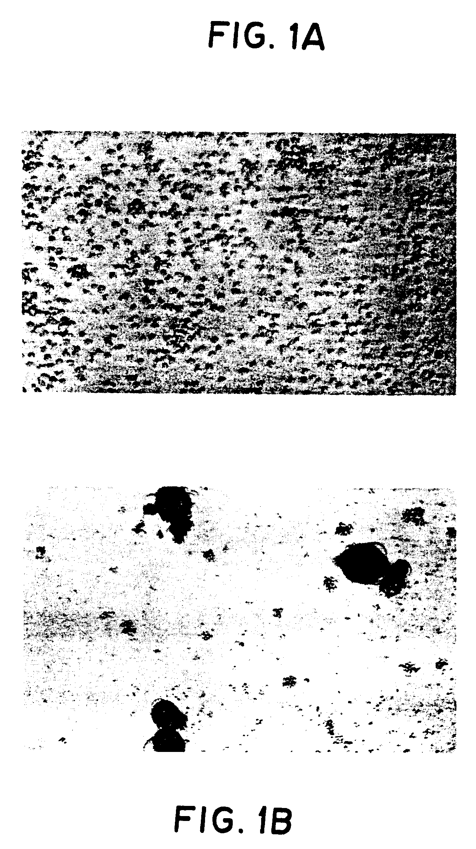

Figure 1 shows syncytia formation in the presence (A) or absence (B) of 320 $\mu$M $\beta$-cyclodextrin sulfate.

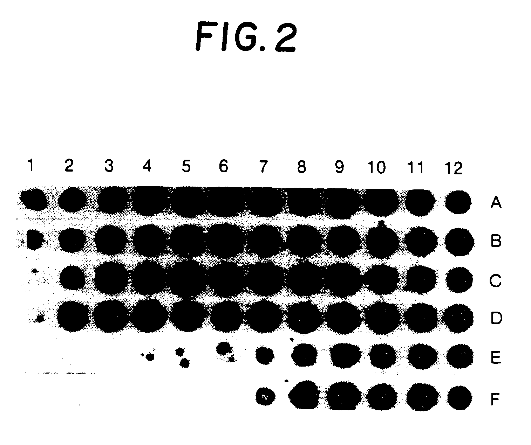

Figure 2 illustrates the effects of cyclodextrin and cyclodextrin derivatives on HIV infectivity as determined by reverse transcriptase activity. Samples assayed are (A) untreated control cells, (B) $\beta$-cyclodextrin unsubstituted ring, (C) partially sulfated $\beta$-cyclodextrin ring, (D) propylated $\beta$-cyclodextrin ring, (E) sulfated $\beta$-cyclodextrin ring, and (F) methylated $\beta$-cyclodextrin ring. Numbers indicate 1/2 dilutions of test compounds with 1 = 2.5 mM concentration of compound.

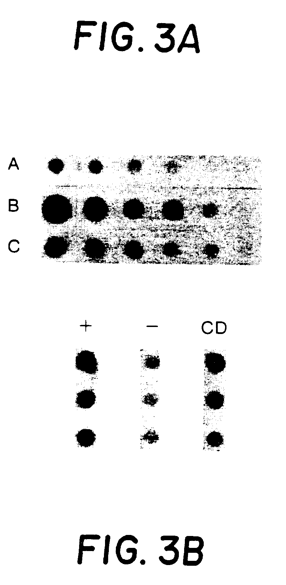

Figure 3 shows the effect of beta-cyclodextrin tetradecasulfate ($\beta$-CD-14S or $\beta$-CD-TDS) on HIV RNA production. Virus particles were incubated with target cells in the presence or absence of 1M of $\beta$-CD-14S. At 72 hours post infection, the cells were washed and dot blot assays were performed to determine the presence or absence of viral RNA production. In (A), target cells were infected in the presence of sulfated $\beta$-cyclodextrin (lane A), in the absence of cyclodextrins (lane B), or in the presence of unsubstituted $\beta$-cyclodextrin (lane C). 1/2 dilutions of cells are from left to right starting with $10^6$ cell/well utilized. In (B), virally infected cells (+) or uninfected cells (-) were assayed for their ability to produce viral RNA by dot blot analysis. CD = infected cells incubated with 1 mM of $\beta$-CD-14S. 1/2 dilutions of cells are from top to bottom starting with $10^6$ cell/well.

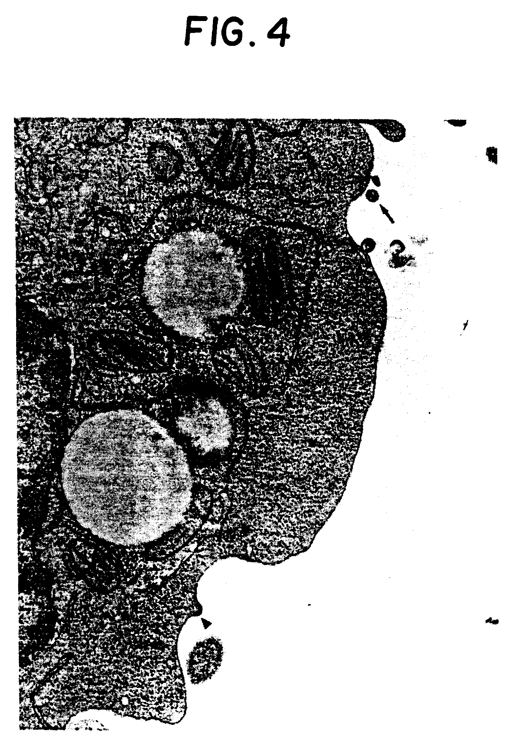

Figure 4 shows an electron micrograph of HIV-1 particles produced by infected H9 cells in the presence of 1 mM of $\beta$-CD-14S. Note the mature morphology of the viral particles produced (upper left) as well as budding virus.

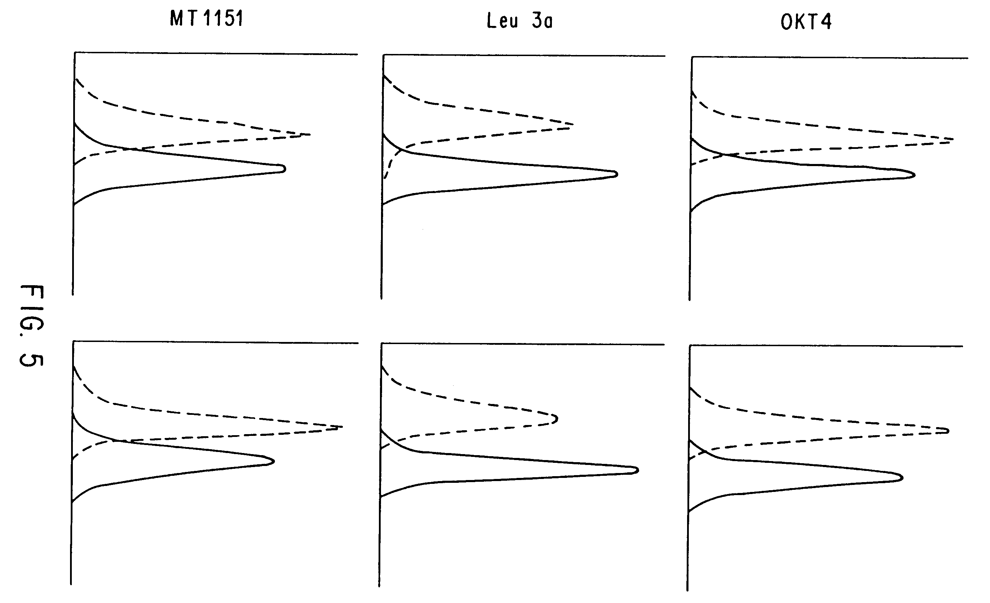

Figure 5 shows monoclonal antibody binding in the presence of 1 $\mu$M of $\beta$-CD-14S. The human T cell line CEM was preincubated with 1 M of $\beta$-CD-14S, and then incubated with various monoclonal antibodies as noted in Section 6.1, infra. Fluorescence histograms show relative fluorescence intensity (abscissa) versus relative cell number (ordinate) for each monoclonal as listed. Primary antibody binding was assessed in the presence (right) or absence (left) of $\beta$-CD-14S.

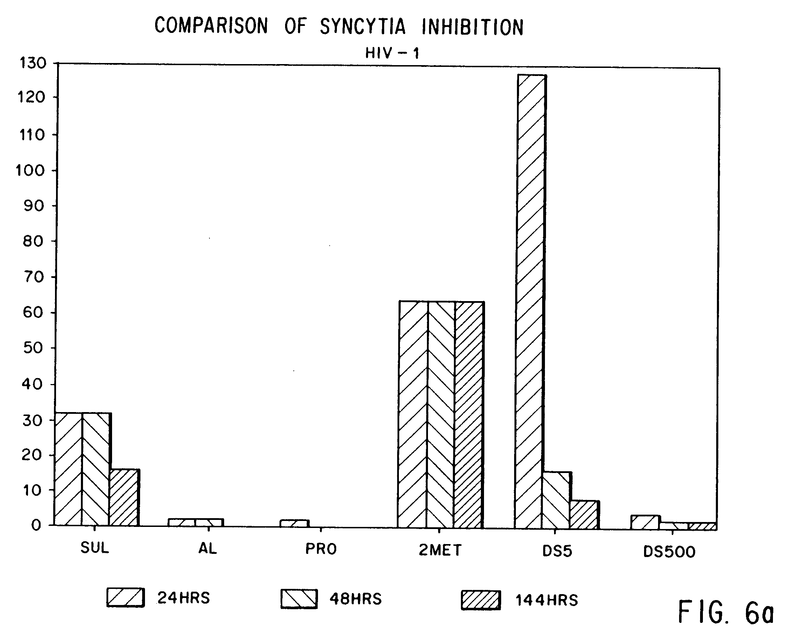

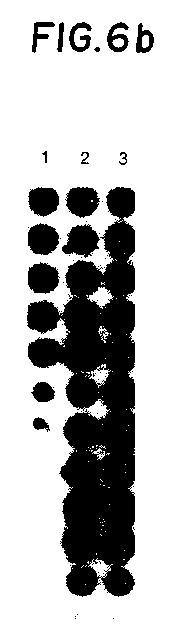

Figure 6 compares the effects of dextran polysulfate and substituted $\beta$-cyclodextrins on HIV infectivity. In (A) degree of syncytia inhibition is shown on the ordinate for each compound added. SUL = 600 $\mu$M $\beta$-CD-14S, AL = 600 $\mu$M unsubstituted $\beta$-CD, Pro = 600 $\mu$M propoxylated $\beta$-CD, 2MET = 300 $\mu$M methoxylated $\beta$-CD, DS5 = 600 $\mu$M dextran sulfate 5,000, DS500 = 600 $\mu$M dextran sulfate 500,000. Results for each of 3 time points are shown. In (B) similar results utilizing reverse transcriptase assays are shown. Infectious virus particles were incubated in the presence of sulfated $\beta$-cyclodextrin (lane 1), dextran sulfate 5000 (lane 2) or media (lane 3) for 6 days at which time cells were resuspended in fresh media and allowed to incubate a further 10 days before being harvested. Reverse transcriptase activity was assayed as described in Section 6.1, infra. 1/2 dilutions of the compound added are shown from bottom (highest concentration at 2.5 mM) to top (lowest concentration).

5

## 5. DETAILED DESCRIPTION OF THE INVENTION

The present invention provides uses for inhibiting infection of cells by a virus which employ a carbohydrate blocking agent capable of interacting with cells to interfere with the binding of the virus with the cells. The invention also relates to using such carbohydrate blocking agents to block cell to cell transmission of the virus. The invention further provides uses for employing such carbohydrate blocking agents to inhibit syncytia formation. The invention further relates to the use of a composition comprising a carbohydrate blocking agent in combination with at least one antiviral agent. Also provided is the use of a composition comprising a carbohydrate blocking agent in combination with at least one steroid or nonsteroidal organic compound where the steroid does not contain glucocorticoid activity.

## 5.1. CARBOHYDRATE BLOCKING AGENTS: OLIGOSACCHARIDES

The carbohydrate blocking agents suitable for use according to the method of the invention comprise one or more very water soluble derivatives of cyclic oligosaccharide having up to twelve sugar monomers. Oligosaccharides are generally defined as carbohydrates containing two up to about ten simple sugars linked together; polysaccharides as having more than ten simple sugars linked together. However, for the purposes herein, the term oligosaccharide is used to mean carbohydrates containing from two to about twelve simple sugars linked together. It is preferred that oligosaccharides having less than about twelve sugar monomers be employed. More preferred cyclic oligosaccharides comprise cyclic molecules having six to eight sugar monomers such as alpha-, beta-, or gamma-cyclodextrin. Cyclodextrins (CDs) are cyclic, non-linear oligosaccharides having at least six glucopyranose units. Although cyclodextrins with up to twelve such units are known, only the first three homologs have been studied extensively. The common designations of the lower molecular weight cyclodextrins are alpha-, beta-, and gamma-cyclodextrin which correspond to 6,7 and 8 glucopyranose units, respectively. Cyclodextrins are also known sometimes as cycloamyloses. A number of cyclodextrin derivatives are known. In general, these chemically modified cyclodextrins are formed by reaction of the primary or secondary hydroxyl groups attached to carbons 2, 3, or 6, without disturbing the alpha (1 - 4) hemiacetal linkages. Sugars other than glucose, such as arabinose, galactose, and other 5- and 6-carbon sugars and many other forms known to those of ordinary skill in the art as "sugars" may be useful.

Highly water-soluble cyclic oligosaccharide derivatives bearing non-ionic and/or ionic substituents are useful according to the present invention. Suitable highly water-soluble cyclic oligosaccharide derivatives include alpha, beta, and gamma CD derivatives having non-ionic substituents including but not limited to alkyl substituents such as methyl, ethyl, etc., as well as those in which a number of hydroxyl groups are replaced by other groups so as to increase the hydrophilic activity of CD. Such groups may include esters, ethers (e.g. alkoxy), thioesters, thioethers, carboxylic acids, carbohydrates, or other groups which convey hydrophilic activity by way of polar or hydrogen bonding constituents. Substitution may occur at some or all of the hydroxyl groups. In a preferred embodiment, the cyclic oligosaccharides contain about 10 to about 16 groups.

The cyclic oligosaccharide derivatives useful in the present invention are highly hydrophilic and therefore very water soluble. A highly hydrophilic character probably is important to allow interaction with cellular surfaces. The hydrophilic activity is likely roughly indicated by the affinity to water, as measured by water solubility. It is important to measure the same at 0°C since at higher temperatures the most suitable derivative have solubilities so high that meaningful measurements are difficult.

It is contemplated that very water-soluble cyclic oligosaccharide derivatives bearing ionic and/or non-ionic substituents may in some instances have advantageous properties, and that these are within the scope of this invention. Although highly water-soluble derivatives in general are believed useful, salt derivatives are preferred. The term "very soluble" as used herein refers to a solubility of at least 20 gm/100 ml measured at 0°C, preferably more than 30 gm/100 ml.

The phrase "salt derivative" as used herein means an ionic compound derived from a cyclic oligosaccharide by reaction with a suitable reagent. The preferred salt derivatives are provided by a cyclic oligosaccharide having substituents selected from the group consisting of sulfate, phosphate, carboxylate and mixtures thereof associated with a nontoxic, physiologically acceptable cation. Many of said preferred derivatives are known compounds. (Croft and Bartech, 1983, Tetrahedron 39:1417-1474). But many potentially useful forms may be variants, structurally or chemically of known compounds. Some of the preferred salt forms of the derivatives are the sodium and potassium forms, since these tend to impart increased water solubility to organic anions. The salt derivatives useful herein will exhibit electrolytic conductivity and osmotic properties characteristic of electrolytes and polyelectrolytes when in aqueous solution. A particularly preferred salt derivative would be a beta cyclodextrin derivative containing about 12-16 sulfate groups. Various degrees of sulfation per glucose unit can be employed, such as an average of one sulfate group per two glucose units or of two sulfate groups per glucose unit. Cyclodextrins having about two sulfate groups per glucose unit are preferred.

The foregoing has been set forth in order to provide a general understanding of the oligosaccharides which are likely useful for the practice of the present invention. It should be understood, however, that such oligosaccharides are described best by what they do rather than by what they are. The oligosaccharides which are useful herein are those oligosaccharides which are capable of interacting with potential host cells in a fashion which interferes with binding of viruses to the host cells. Those skilled in the art will recognize that the manner of interference with binding of a virus to a host cell will vary according to the particular virus-host cell binding which is involved. Different portions of the oligosaccharides are expected to be involved in binding to potential host cells according to the specific requirements of a particular virus-host cell interaction.

## 5.2. ANTIVIRAL AGENTS

According to one embodiment of the invention, a very water soluble cyclic oligosaccharide derivative in combination with at least one antiviral agent is employed to interfere with virus interaction with a cell. An antiviral agent is any organic or inorganic compound that inhibits any stage of the viral replication cycle including but not limited to the binding of the virus to the cell, entry of the virus into the cell, uncoating of the virus, inhibition of the replication of the viral nucleic acid, inhibition of viral protein synthesis, and maturation of the virus particle.

An example of an antiviral agent is a nucleoside derivative. Nucleoside derivatives are modified forms of the purine (adenosine and guanosine) and pyrimidine (thymidine, uridine, and cytidine) nucleosides which are the building blocks of RNA and DNA. The nucleoside derivatives which can be utilized in the currently claimed invention include but, are not limited to: AZT (3'-azido-2',3'-dideoxythymidine, azidothymidine, zidovudine);

2',3' dideoxynucleosides including 2',3' dideoxyadenosine, 2',3' dideoxyguanosine, 2',3' dideoxycytidine, 2',3' dideoxythymidine, and 2',3' dideoxyinosine;

3'-deoxythymidine-alene (3'-deoxy-2',3'-didehydrothymidine; and

D4T (2',3'-didehydro-2',3'-dideoxythymidine).

Other examples of antiviral agents include but are not limited to a CD4 peptide or analog thereof and hypericin.

## 5.3. STEROIDS AND NONSTEROIDAL ORGANIC COMPOUNDS

The present invention provides for any composition which comprises a cyclic oligosaccharide derivative alone or in combination with a steroid or non-steroid organic compounds that are cell growth modulators, i.e. a compound that is capable of altering or changing growth behavior, including both promotion and inhibition.

Most preferred among the steroid growth modulating compounds are those latent growth-inhibiting steroids which lack glucocorticoid and mineralo-corticoid activity, since such activity is an undesired side effect and limits the dose size or extent of use of the steroid for the purpose of the present invention. Among such more preferred steroids are 11 alpha,17,21-trihydroxypregn-4-ene-3,20-dione (11-desoxycortisol or cortexolone), and tetrahydrocortisol.

Non-steroid organic compounds may include, but are not limited to compounds which may be peptide, carbohydrate, or lipid in nature. Examples of such compounds may include but are not limited to interleukin-1, interleukin-2, and interferons alpha, beta, and gamma.

## 5.4. APPLICATIONS AND USE

The present invention provides novel uses for inhibiting infection of cells by a virus. A carbohydrate blocking agent capable of interacting with host cells is provided to interfere with binding of the virus with the cells. The carbohydrate blocking agent comprises one or more oligosaccharides having up to about twelve sugar monomers. The host cells are contacted with the carbohydrate blocking agent in an amount, for a time and under conditions selected to effect interaction of the carbohydrate blocking agent with the cells to result in the interference with binding of the virus with the cells.

The carbohydrate blocking agent is administered to the cells at a concentration effective to interfere with binding of the virus to the cells. The concentration of any particular oligosaccharide agent administered to cells will depend on such parameters as its solubility in the medium surrounding the cells, its ability to interfere with binding of a virus to the potential host cells and its effects on other biological systems of the cell. It is expected that concentrations ranging from approximately 0.15 to 2 millimolar will result in interference with binding of the virus to the cells. The carbohydrate blocking agents is administered to the cells for a time sufficient to allow and maintain interference of binding of the virus to the cells. Those skilled in the art will recognize that the length of time that a particular carbohydrate blocking agent will be administered is dependent on such factors as concentration of carbohydrate blocking agent administered, the type of virus-host cell interaction and the ability of the carbohydrate blocking agent to interfere with binding of the virus to the cells.

Conditions under which a particular carbohydrate blocking agent will effect interference with binding of the virus to potential host cells may be determined with suitable assays such as the syncytia inhibition assay described in Section 6.11, infra. Such assays will help in determining the concentrations of the carbohydrate blocking agent which are most effective in inhibiting infection of cells with the virus.

Those skilled in the art will recognize that concentrations of the carbohydrate blocking agents may be presented to cells within the body of a mammal, including humans, by any of several routes of administration using dosages which provide at least the above mentioned concentrations in the extracellular medium of the body at the cell surfaces. Such routes of administration include, inter alia, oral, intravenous, and transdermal routes.

The methods of the invention are suitable for use to treat infection by viruses. They may be used with retroviruses, including but not limited to human immunodeficiency virus-1 (HIV-1), human immunodeficiency virus-2 (HIV-2), human T-cell lymphotropic virus-I (HTLV-I), human T cell lymphotropic virus (HTLV-II), avian leukosis virus, Rous sarcoma virus, avian erythroblastosis virus, avian myeloblastosis virus, feline leukemia virus, and bovine leukemia virus. They may also be used with paramyxoviruses (e.g. measles virus and respiratory syncytial virus) and herpesviruses (e.g. Epstein Barr virus). Such compounds may act to interfere with binding of the virus with the cells. Such cells may include but are not limited to lymphoblastoid cells (T cells and B cells) and monocytes.

The inhibition of binding of a virus to a potential host cell is believed to be important in preventing infection of the cell with the virus. In the case of a retrovirus such as HIV-1 or HIV-2, attachment and entry into a host cell are important parts of the life cycle of the virus. If the retrovirus is prevented from entering a cell, the cell will not become infected with the virus. One of the consequences of infection with a virus such as a retrovirus and more particularly HIV-1, is that the cells begin to fuse together forming syncytia. The formation of syncytia is lethal to the affected cells.

It is further believed that some cell surface receptors are also enzymes which recognize certain extracellular substances having short polysaccharide residues. Some of these cell surface receptors are believed to be glycosyltransferases which recognize short polysaccharide residues present in glycoproteins and other biological molecules. It is believed that glycoproteins found on viral surfaces and through which viruses manifest specific cell type or species specific tropisms also use such cell surface receptors/enzymes to bind the host cells prior to infection of the cells.

Prevention of interaction between the virus and potential host cell is important in minimizing the pathophysiology of disease where syncytia formation is an important part of the action of the virus or viral agent in the disease process.

The invention also provides uses for inhibiting cell to cell transmission of a virus. A carbohydrate blocking agent capable of interacting with host cells is provided to interfere with the transmission of virus from one cell to another cell. Examples of such cells include but are not limited to T cells; B cells, and monocytes. The host cells are contacted with the carbohydrate blocking agent in an amount, for a time and under conditions selected to effect interaction of the carbohydrate blocking agent with the cells to result in the interference with the transmission of the virus from cell to cell. The carbohydrate blocking agent is a very water soluble cyclic oligosaccharide derivative. These uses are suitable for use with retroviruses including but not limited to human immunodeficiency virus-1 (HIV-1), human immunodeficiency virus-2 (HIV-2), human T-cell lymphotrophic virus-I (HTLV-1), human T-cell lymphotrophic virus-II (HTLV-II), avian leukosis virus, Rous sarcoma virus, avian myeloblastosis virus, feline leukemia virus, and bovine leukemia virus. These uses may also be used with paramyxoviruses (e.g. measles virus and respiratory syncytial virus) and herpesviruses (e.g. Epstein Barr virus).

The invention also provides uses for inhibiting formation of syncytia between cells. A carbohydrate blocking agent which prevents the interaction of the virus with the cell is provided. Examples of such cells include but are not limited to T cells, B cells, and monocytes. The carbohydrate blocking agent is administered to the cells under conditions selected to allow the agent to interact with the cells, thus inhibiting formation of syncytia. The carbohydrate blocking agent in the present invention is a very water soluble cyclic oligosaccharide derivative. These uses are suitable for use with retroviruses, including but not limited to human immunodeficiency virus-1 (HIV-1), human immunodeficiency virus-2 (HIV-2), human T cell lymphotrophic virus-I (HTLV-1), human T-cell lymphotrophic virus-II (HTLV-II), avian leukosis virus, Rous sarcoma virus, avian myeloblastosis virus, feline leukemia virus, and bovine leukemia virus. These uses may also be used with paramyxoviruses (e.g. measles virus and respiratory syncytial virus) and herpesviruses (e.g. Epstein Barr Virus).

The effectiveness of the carbohydrate blocking agents on virus infection, cell to cell transmission of the virus, and syncytia formation may be assayed using procedures known in the art. A description of such procedures is given in Section 6.1, infra.

The invention further provides the use of compositions of very water soluble cyclic oligosaccharide derivatives described in Section 5.1, supra in combination with antiviral agents described in Section 5.2, supra. Such compositions may be used to inhibit the binding of a virus to a cell. In another embodiment, the compositions may be used to inhibit cell to cell transmission of the virus. In a further embodiment, such compositions may be used to inhibit syncytia formation. These compositions may be used with retroviruses, such as human immunodeficiency virus-1, human immunodeficiency virus-2, human T-cell lymphotrophic virus-I (HTLV-1), human T-cell lymphotrophic virus-II (HTLV-II), avian leukosis virus, Rous sarcoma virus, avian myeloblastosis virus, feline leukemia virus, and bovine leukemia virus. Additionally, these compositions may be used with paramyxoviruses (e.g. measles viruses or respiratory syncytial virus) or herpesviruses (e.g. Epstein Barr Virus). The effectiveness of the compositions of the present invention on virus infection or syncytia formation, may be assayed using procedures known in the art. A description of such procedures is given in Section 6.1., infra.

The invention further provides the use of compositions of very water soluble cyclic oligosaccharide derivatives described in Section 5.1, supra. Such compositions may be used to inhibit the binding of a virus to a cell in combination with steroid or non-steroid organic compounds that contain growth modulating activity described in Section 5.3, supra. These compositions may be used with retroviruses, such as human immunodeficiency virus-1, human immunodeficiency virus-2, human T-cell lymphotrophic virus-I (HTLV-1), human T-cell lymphotrophic virus-II (HTLV-II), avian leukosis virus, Rous sarcoma virus, avian myeloblastosis virus, feline leukemia virus, and bovine leukemia virus. Additionally, these compositions may be used to treat Kaposi's Sarcoma which is often manifested in AIDS patients. The effectiveness of the compositions of the present invention on virus infection or syncytia formation, may be assayed using procedures known in the art. A description of such procedures is given in Section 6.1, infra.

The invention further provides the use of compositions of very water soluble cyclic oligosaccharide derivatives described in Section 5.1, supra. Such compositions may be used to inhibit the binding of a virus to a cell in combination with steroid or non-steroid organic compounds that contain cell growth modulating activity described in Section 5.3 and antiviral agents described in Section 5.2, supra. These compositions may be used with retroviruses, such as human immunodeficiency virus-1, human immunodeficiency virus-2, human T-cell lymphotrophic virus-I (HTLV-1), human T-cell lymphotrophic virus-II (HTLV-II), avian leukosis virus, Rous sarcoma virus, avian myeloblastosis virus, feline leukemia virus, and bovine leukemia virus. Additionally, these compositions may be used to treat Kaposi's Sarcoma which is often manifested in AIDS patients. The effectiveness of the compositions of the present invention on virus infection or syncytia formation, may be assayed using procedures known in the art. A description of such procedures is given in Section 6.1, infra.

## 6. EXAMPLE: SYNTHETIC CYCLODEXTRINS INHIBIT HIV INFECTION IN VITRO

The following examples are offered by way of illustration and not by way of limitation. The invention has been described herein with reference to certain preferred embodiments and examples. However obvious variations will be apparent to those skilled in the art. The invention is not to be considered limited to the particularly exemplified embodiments.

## 6.1. MATERIALS AND METHODS

The fusion inhibition (syncytia) assay was performed according to published procedures (Dalgleish et al., Nature 312:763, (1984) and Sodroski et al., Nature 322: 470, (1986)). Sup-T1 cells are favored target cells for their rapid degree of cell fusion when co-cultured with HIV-1 producing cell lines. Cell culture is performed according to the method of Dalgleish et al. (Nature 312:763, (1984)). Sup-T1 cells are plated in

96 well plates ($10^5$ cells/well in RPMI 1640 + 10% FCS) and incubated with or without dilutions of beta-cyclodextrin tetradecasulfate ($\beta$-CD-TDS or $\beta$-CD-14S) at 37°C. HTLV-III B (HIV-1) or WMJ infected H9 cells are then added $5\times10^4$/well and the number of multinucleated giant cells per 16 X field counted with a Zeiss inverted field phase contrast microscope after 18 hours. Syncytia are easily identified and inhibition of syncytia by different dilutions of $\beta$-CD-TDS can be compared with to $\beta$-CD-TDS free preparations. It will be apparent to those skilled in the art, that the formation of syncytia between cells susceptible of infection by HIV-1 and cells expressing HIV-1 envelope glycoproteins represents the same molecular phenomena involved in infection by HIV-1, but is far less hazardous and may be employed to study the mechanisms of infection and the effects of modulating factors on HIB-1 intracellular infection mechanisms, without fear of exposure to the virus itself.

For screening of different dilutions, 10-fold dilutions of a 150 micromolar solution of $\beta$-CD-TDS were prepared and directly added to syncytia assays as described above.

### 6.1.1. SYNCYTIA ASSAYS

For the assay of HIV-1 fusion, Supt T1 cells were used as target cells. They fuse rapidly when cocultured with HIV-1 (H9/IIIb) producing cell lines. For the assay of HIV-2 mediated cell fusion, H9 cells were used as target cells. They fuse rapidly when cocultured with HIV-2 (CEMROD-2) producing cell lines. HIV infected cells were plated in 96 well plates ($10^4$ cells/well in RPMI 1640 + 10% FCS) and incubated with or without dilution of compounds for 30 minutes at 37°C. Target cells are then added $5 \times 10^5$/well and the number of syncytia are qualitatively determined after the incubation periods described in the text.

### 6.1.2. REVERSE TRANSCRIPTASE ASSAY

Virus infected cells were pelleted by centrifugation and 50 $\mu$l of culture suprenatant was collected from each well for assay. Culture supernatant (25 $\mu$l) was plated in 96 well round bottomed plates and 50 $\mu$l of transcriptase cocktail was added to each well. Cocktail is 50 mM Tris-HC1, pH 8.0, 20 mM DTT, 0.6 mM MnC1$_2$, 60 mM NaC1, 0.05% NP-40, 5 $\mu$g/ml dt (oligodeoxythymidilic acid), 10 $\mu$g/ml poly A (polyriboadenylic acid), 10 $\mu$M dTTP, 1 Ci/mm $^{32}$P dTTP. Plates were then incubated for 60 minutes at 37°C. After incubation samples were spotted onto Whatman DEAE-81 paper, washed and analyzed by counting or direct autoradiography.

### 6.1.3. VIRAL RNA ANALYSIS

Virus treated cells were grown in tissue culture and harvested for assay at 72 hrs. post-infection. The cell concentrations were then adjusted to $1 \times 10^6$ cells/m1 with phosphate buffered saline and spotted directly onto prewetted gene screen utilizing a dot blot apparatus. The filter was then fixed in 3% NaC1, 10 mM NaH$_2$PO$_4$, (pH 7.2) containing 1% gluteraldehyde at 4°C for 1 hr. The fixed blot was then rinsed three times with proteolytic buffer (50mM EDTA, 0.1 M Tris-HC1 (pH 8.0), 20 $\mu$g/ml proteinase K) for 30 minutes at 37°C. The filter was air dried until being probed. A Sall-Xho1 probe derived from the pIIIenv1 plasmid (a generous gift of Dr. J. Sodrofsky) and encoding the envelope region of the HXB2, HIV-1 virus was labeled by the random primer method using standard technology and hybridized to the filter as previously described (Weiner et al., 1986, Cell Immunol. 100:197).

### 6.1.4. STRUCTURAL STUDIES

Virus infected cells were washed and resuspended in fresh media in the presence or absence of test compounds. After a 48 hour culture period the cultures were pelleted and fixed in 1% glutaraldehyde, 0.1 M Cacodylate-HC1, pH 7.3 followed by OsO$_4$, and embedded in spur resin for examination of these sections by electron microscopy.

### 6.1.5. CYTOFLUORIMETRY

Human T cell lines were centrifuged and washed 2X in FACS buffer (1% bovine serum albumin in phosphate buffered saline with 0.1% NaN$_3$). The cells were resuspended at $10^7$/ml and preincubated with or without $\beta$-CD-TDS as indicated. Primary antibody was then added for an additional 30 minutes, and the samples prepared as previously described (Williams et al., 1988, Proc. Natl. Acad. Sci. U.S.A. 85:6488).

6.2. RESULTS

6.2.1. INHIBITION OF HIV INFECTIVITY BY SUBSTITUTED β-CYCLODEXTRINS

A prominent feature of the human lentiviruses is their ability to fuse with target cells. When analyzed in vitro on human target cell lines, HIV-mediated infection results in the formation of multinucleated giant cells or syncytia. Such syncytia have been observed to contribute to the pathology of immune cell depletion characteristic of the infection observed in AIDS patients. This fusion process differs from the mechanism of cell entry of many other retroviruses in that fusion does not require the acidic pH of endocytotic vesicles. In vitro HIV-mediated cell fusion is thought to occur in an analogous way to virus entry in vivo.

In a series of preliminary experiments, a syncytia assay induced by HIV-1 fusion of Sup-T1 cells was performed as described in Section 6.1.1, supra. Results are illustrated in Table 1.

As shown in Table 1, at a range of concentrations of β-CD-TDS between 1.5 micromolar and 1500 micromolar, inhibition of syncytia was observed. In contrast, no inhibition of syncytia formation was observed at concentrations of 0.15 micromolar.

## TABLE 1

### Syncytia Inhibiting Activity: Degree of Syncytia Formation[a]

| | Concentration of β-CD-TDS (micromolar) | | | | |
|---|---|---|---|---|---|
| Viral Strain | 1500 | 150 | 15.0 | 1.50 | 0.15 |
| HTLV III -B (HIV-1 IIIB) | 0 | 2M | 4L | 4L | 4L |
| HTLV III WMJ | 0 | 0 | 1S | 3M | 4L |

a  Degree of syncytia formation        Size of syncytia formed
   4=Full                             S=Small
   0=None                             M=Medium
                                      L=Large

In a series of additional experiments, analysis of HIV-1 induced syncytia was performed by coculturing H9 cells infected with the IIIb isolate with uninfected Supt-1 (a human CD4+ T cell line) cells as targets. For the assay of HIV-2, HUT 78 cells infected with the ROD-2 isolate were cocultured with H9 (a human CD4+ T cell line) target cells. β-cyclodextrin with no substituents (β-CD), with 4 sulfate groups (β-CD-4S), and with 4 propoxy groups (β-CD-4Pr) were as ineffective as media alone in inhibiting syncytia formation of HIV-1. In contrast, β-cyclodextrin bearing 14 sulfate groups per molecule (β-CD-TDS) mediated extremely high antisyncytia activity as shown in Table 2 and Figure 1.

TABLE 2

| Ability of Cyclodextrins to Block HIV-1 Entry[a] | | | | | | | | | | |
|---|---|---|---|---|---|---|---|---|---|---|
| Compounds[b] | Solubility 0°C g/100ml $H_2O$ | Concentration (mM) $\beta$-Cyclodextrins | | | | | | | | |
| | | 2.5 | 1.3 | .63 | .32 | .16 | .08 | .04 | .02 | .01 |
| Control | | 4L | 4L | 4L | 4L | 4L | 4L | 4L | 4L | 4L |
| Beta-CD | 0.7 | 4L | 4L | 4L | 4L | 4L | 4L | 4L | 4L | 4L |
| Beta-CD-Pr | 20 | 1m | 4L | 4L | 4L | 4L | 4L | 4L | 4L | 4L |
| Beta-CD-4-7S | 13 | 3m | 4L | 4L | 4L | 4L | 4L | 4L | 4L | 4L |
| Beta-CD-14S | 42 | 0 | 0 | 0 | 0 | 1s | 2m | 3L | 4L | 4L |
| Beta-CD-M | 32 | 0 | 0 | 0 | 0 | 0 | 0 | 3m | 4L | 4L |

a Results are expressed as the number of syncytia formed: Syncytia are scored numerically from 0 to 4, with 4 being multiple syncytia widely distributed and 0 indicating no syncytia observed. The size of the syncytia is recorded as s = small, m = medium and L = large syncytia.
b Beta-CD is unsubstituted cyclodextrin ring, Beta-CD-Pr is basic ring structure substituted with propoxy groups, Beta-CD-14S is basic ring structure completely substituted with sulfates, Beta-CD-M is basic ring structure completely substituted with methoxy groups.

$\beta$-cyclodextrin with 14 methoxy group ($\beta$-CD-14Me) exhibited a greater degree of syncytia inhibition than was observed with B-CD-TDS. The inhibition of syncytia formation was observed at doses below 80 $\mu$M.

Similar results for syncytia induction mediated by the ROD-2 isolate of HIV-2 were obtained as shown in Table 3.

TABLE 3

| Ability of Cyclodextrins to Block HIV-2 Entry[a] | | | | | | | | | | |
|---|---|---|---|---|---|---|---|---|---|---|
| Compounds[b] | Solubility 0° gm/100ml $H_2O$ | Concentration of (mM) of $\beta$-Cyclodextrin | | | | | | | | |
| | | 2.5 | 1.3 | .63 | .32 | .16 | .08 | .04 | .02 | .01 |
| Control | | 4L | 4L | 4L | 4L | 4L | 4L | 4L | 4L | 4L |
| Beta-CD | 0.7 | 4L | 4L | 4L | 4L | 4L | 4L | 4L | 4L | 4L |
| Beta-CD-14S | 42 | 0 | 0 | 0 | 1s | 2m | 3L | 4L | 4L | 4L |
| Beta-CD-M | 32 | 0 | 0 | 0 | 0 | 0 | 0 | 3m | 4L | 4L |

a Results are expressed as the number of syncytia formed: Syncytia were scored numerically from 0 to 4, with 4 being multiple syncytia widely distributed and 0 indicating no syncytia observed. The size of the syncytia is recorded as s = small, m = medium and L = large syncytia.
b Beta-CD is unsubstituted cyclodextrin ring, Beta-CD-14S is basic ring structure completely substituted with sulfates, Beta-CD-M is basic ring structure completely substituted with methoxy groups.

Both polysulfated $\beta$-CD and poly-methoxylayted $\beta$-cyclodextrin inhibited syncytia formation mediated by ROD-2. However, the degree of inhibition was substantially greater with $\beta$-CD-14Me.

The ability of cyclodextrins to inhibit HIV infection was assessed in vitro utilizing reverse transcriptase assays as described Section 6.1.2, supra. HTLV-IIIb cell free virus stocks were inoculated onto target cells in the presence or absence of the synthetic compounds. After a 10 day incubation period, wells were harvested and reverse transcriptase activity scored (Figure 2). $\beta$-Cyclodextrin itself (lane B) exhibited no inhibition of viral infectivity as reverse transcriptase activity appeared similar to the positive infectivity control (lane A). Partially sulfated (lane C) and propoxylated $\beta$-cyclodextrin (lane D) were in essence ineffective, with minimal inhibition of reverse transcriptase activity. In contrast, the highly sulfated $\beta$-CD-14S was extremely effective in inhibiting in vitro infectivity (lane E). While some activity was apparent at concentrations as high as 320 $\mu$M (dilution 4), reverse transcriptase activity was dramatically inhibited at concentrations as low as 80 $\mu$M (dilution 6). The $\beta$-CD-14Me (lane F) was at least as effective, with no viral reverse transcriptase activity apparent in cultures from cells in contact with virus in the presence of doses

12

as low as 80 $\mu$M to 40 $\mu$M (dilution 7). While these results are similar to those from syncytia assays, they suggest that viral infectivity in the presence of low amounts of some substituted cyclodextrins is markedly decreased even at concentrations which do not fully inhibit syncytia formation.

6.2.2. SITES OF ACTION OF CYCLODEXTRINS

In order to determine the specific sites of viral pathogenesis interrupted by the cyclodextrin compounds, we assayed the ability of the cyclodextrins treatment to effect reverse transcriptase levels of cells already infected with HIV-1. In contrast to the results observed when virus was added to uninfected cells, (see supra, Section 6.2.1.) no significant inhibition of reverse transcriptase was observed. This suggests that the cyclodextrins must exert their effects at earlier stages of the virus life cycle.

To determine if the cyclodextrins were interfering with steps relevant to viral replication, the ability of these compounds to inhibit viral nucleic acid synthesis was determined. Virus particles were incubated with target cells in the presence or absence of 1 mM of $\beta$-CD-14S. At 72 hours post-infection, the cells were washed and dot blot assays were performed to determine the presence or absence of viral RNA production. From target cells infected in the presence of sulfated $\beta$-cyclodextrin (Figure 3A, lane A) little or no HIV-1 specific RNA was detected. In both the unsubstituted ring and the positive control lane, substantial viral RNA was detected at this same time point. However, the inhibition of viral nucleic acid replication was not a direct effect on the replicative machinery of the virus. Infected cells incubated with sulfated $\beta$-cyclodextrin were not inhibited in their ability to produce viral RNA (Figure 3B). These results complement syncytial and reverse transcriptase assays suggesting that the antiviral effects mediated by these compounds operate early in the viral attack on the cell.

While substituted $\beta$-cyclodextrins do not inhibit viral RNA or reverse transcriptase activity in infected cells, it is possible that these compounds interfere with viral maturation. To further address this point, we analyzed virus particle production in the presence of specifically substituted cyclodextrins by electron microscopic examination. Virus infected cells were washed and resuspended in fresh media in the presence or absence of test compounds. After a 48 hour culture period, the cultures were pelleted and fixed in 1% glutaraldehyde, 0.1 M Cacodylate-HC1, pH 7.3 followed by OsO$_4$ and embedded in spur resin for examination of thin sections.

Virus preparations treated in the presence of 300 $\mu$M sulfated cyclodextrins demonstrated membrane dense regions corresponding to virus budding and shedding. The HIV morphology observed in this shed virus was typical of the lentivirus family (Figure 4). Detailed morphological examination of viral particles produced in the presence of substituted cyclodextrins exhibited normal mature HIV phenotype. Additionally, we observed no significant deviation in the ratio of mature virus present after cyclodextrin treatment or in the total number of virus particles produced in the presence of these compounds. Together, these results suggest that substituted $\beta$-cyclodextrins exert no direct effects on HIV virus production or maturation. They apparently exert their effects at the cell membrane during the initial phases of attack on the cell.

6.2.3. INFLUENCE OF CYCLODEXTRINS ON INTERACTIONS WITH CD4

Both HIV-1 and HIV-2 infected cells express the CD4 human T cell surface antigen. The strict tropism observed is a manifestation of high affinity binding sites displayed on the retrovirus envelope glycoprotein which mediates binding to CD4 bearing cells. The ability of some fully substituted cyclodextrin molecules to specifically interfere with syncytia formation, reverse transcriptase activity, and the transfer of infectious viral RNA is similar to the effects of antibodies directed at the CD4 molecule. A subset of anti-CD4 antibodies specifically disrupt the viral envelopes' ability to interact with this specific receptor. A possible mode of action of these compounds is interaction with these specific sites on CD4. We have therefore determined the ability of 1 mM substituted $\beta$-cyclodextrin to interfere with the binding of monoclonal antibodies directed at different epitopes of the CD4 glycoprotein. Flow cytometry was utilized for this investigation.

The Leu3a monoclonal antibody binds to the first cysteine bounded region of the CD4 glycoprotein and has been demonstrated to directly interfere with HIV-1, HIV-2 as well as SIV binding. The MT1151 monoclonal antibody has been mapped to the second cysteine bounded region of CD4 and is implicated in secondary structural constraints necessary for gp120-CD4 interactions. The OKt4 monoclonal antibody binds to a region distal to these first two monoclonal antibodies and is mapped to a region that is not necessary for the binding reaction between HIV virus and the CD4 positive cell.

A comparison of the reactivity patterns of these antibodies on treated and control cells demonstrates that within the limits of this analysis, none of the epitopes recognized by these monoclonal antibodies was decreased in its ability to interact with CD4 (Figure 5). These results suggest that the protein interactions important for binding to CD4, in an analgous way to virus binding, are not altered by the cyclodextrin treatment. Furthermore, the results also suggest that these compounds do not interfere with the expression of CD4 on the target cells. While these results do not rule out an effect on gp120-CD4 interactions, they indicate cyclodextrins have no direct effect on the specific protein-protein interactions at the CD4 site.

## 6.2.4. COMPARISON OF SUBSTITUTED CYCLODEXTRINS WITH DEXTRAN POLYSULFATES

Recent studies demonstrated the ability of the polyanionic dextran sulfates to inhibit HIV-1 infection in vitro (Mitsuya et al., 1988, Science 240:646-649 and Baba et al., 1988, Proc. Natl. Acad. sci. U.S.A. 85:6132-6136. The inhibition of virus activity observed with substituted $\beta$-cyclodextrins appears similar to those reported for the dextran sulfates. We have assessed and compared the effects of these compounds and substituted $\beta$-cyclodextrins on HIV-induced syncytia formation (Figure 6A).

At 24 hours, fully sulfated $\beta$-cyclodextrins and fully methylated $\beta$-cyclodextrin exhibited similar ability to block HIV-1 infection as dextran sulfate 5000. All these compounds were more effective than the larger dextran sulfate 500,000 at blocking syncytia formation. However, there was a dramatic difference in the length of the anti-viral effect evidenced by the two classes of complex sugars (Figure 6A & B). By 48 hours, the activity of dextran sulfate 5000 had decreased by almost 90%. In the cultures treated with the methylated or sulfated cyclodextrins, the anti-syncytial activity remained unchanged during the culture period. This result became more pronounced by 144 hours in culture at which point there is little or no antiviral effect of the dextran sulfate 5000 even when present in the several hundred micromolar range. In contrast, the substituted $\beta$-cyclodextrins lost only 50% of their potency following 6 days in continuous culture.

These results have been confirmed utilizing reverse transcriptase assay as specific indicator of viral entry and infectivity (Figure 6B). Infectious virus particles were incubated in the presence of sulfated $\beta$-cyclodextrin (lane 1), dextran sulfate (lane 2) or media (lane 3) for 6 days at which time cells were resuspended in fresh media and allowed to incubate a further 10 days before being harvested and reverse transcriptase activity assayed. The sulfated $\beta$-cyclodextrinmediated significant anti-viral activity even after prolonged periods of tissue culture. This result may be related to susceptibility differences between dextran sulfate and the synthetic $\beta$-cyclodextrin to enzymatic degradation during tissue culture.

## 6.3 DISCUSSION

We have demonstrated dramatic antiviral effects of carbohydrate viral blocking agents, particularly fully substituted sulfated or methylated $\beta$-cyclodextrins. These antiviral effects are intimately associated with the sidechains presented by the cyclodextrin ring, and are not a function of the ring itself. Furthermore, we have examined the effect of several differently substituted molecules and have concluded that only a specific subgroup of sidechains are able to mediate significant anti-HIV effects. These compounds appear to interfere with an early event associated with HIV pathogenesis. There are several lines of evidence for these conclusions. The fully sulfated $\beta$-cyclodextrins exhibit no detectable effects on cells that have already been infected with HIV-1. We have observed no effects on reverse transcriptase activity, virus particle production, or virus particle morphology from infected cell lines incubated with concentrations of the cyclodextrins shown to be protective in syncytia assays i.e., prior to virus infection of cells. In contrast, cyclodextrin, at equivalent concentrations, blocks HIV infection of uninfected cells as demonstrated by reverse transcriptase assays and the presence of viral RNA, in addition to syncytia formation. Together, these data support a model of interference with viral function early in the encounter between virus and cell.

We have tested the ability of these compounds to specifically inhibit monoclonal antibody binding to the known receptor for HIV. The results demonstrate that there was no significant inhibition of antibodies that specifically bind to regions of CD4 necessary for viral function. This finding is important as it underscores the fact that treatment of human cells with these compounds is not likely to effect the function of this immunologically important receptor.

Several studies have indicated a general affinity for absorption (adhesion) to cell surfaces by highly hydrophilic glycosaminoglycans and sulfated saccharides, including cyclodextrin polysulfates (Folkman et at., 1989, Science 243:1490-1493 and Folkman and Weisz, 1989, Amer. Chem. Soc. Symp. Ser. 392: 19-32) in a variety of circumstances (Folkman et al., 1989, Science 243:1490-1493; Folkman and Weisz, 1989, Amer. Chem. Soc. Symp. Ser. 392:19-32; Mitsuya et at., 1988, Science 240:646-649; Ito et at., 1987, Antiviral Res. 7:361-367; DeClerq, 1986, J. Med. Chem. 29:1561-1569; Cole et al., 1986, Nature 323:723; Hook et al., 1984, Ann. Rev. Biochem. 53:847-869; and DeSomer et al., 1968, J. Virol 2:886-893). In view of this, our results suggest that strong attachment of the agents to the cell membrane and/or the viral envelope resulting in reduced diffusion rates of receptors, thereby critically effecting the kinetics of intermolecutar interactions between specific virus ligand and cell receptor molecules, by virtue of classical physical chemical principles (Lauffenburger and DeLisi, 1983, Internat. Rev. Cytol. 84:268-302).

We have compared dextran polysulfates and substituted β-cyclodextrin molecules in HIV-1 syncytia and reverse transcriptase assays. We have observed similar effects from both classes of compounds, but find a unique difference in the longevity of the antivirat effects mediated by cyclodextrins as opposed to dextran sulfates. The results indicate that the substituted cyclized compound may be more stable to prolonged contact with cells and suggests a potential for these compounds to be effective with less frequent administration without diminishing the desired antiviral effects. Finally, the observation that this class of compound interferes with early events of lentivirus pathogenesis suggests that these compounds should be useful in the treatment of other human and animal pathogens with similar mechanisms of entry.

**Claims**

1. Use of a carbohydrate blocking agent for the preparation of a medicament for inhibiting infection of cells by a virus which is capable of interacting with said cells or virus to interfere with binding of the virus with the cells, said carbohydrate blocking agent comprising a cyclic oligosaccharide derivative having up to about twelve sugar monomers, wherein said derivative is characterized by a solubility at O°C in distilled water of at least 20 gm/100 ml.

2. The use according to claim 1, wherein said cyclic oligosaccharide is alpha-, beta-, or gamma-cyclodextrin.

3. The use according to claim 1, wherein said cyclic oligosaccharide derivative is an anionic salt derivative of said oligosaccharide having substituents selected from the group consisting of sulfate, phosphate, carboxylate, and mixtures thereof associated with a physiologically acceptable cation.

4. The use according to claim 3, wherein said cyclic oligosaccharide is beta-cyclodextrin tetradecasulfate.

5. The use according to claim 1, wherein said virus is a retrovirus.

6. Use of a carbohydrate blocking agent for the preparation of a medicament for inhibiting cell to cell transmission of a virus which is capable of interacting with said cells or virus to interfere with binding of the virus with the cells, said carbohydrate blocking agent comprising a cyclic oligosaccharide derivative having up to about twelve sugar monomers, wherein said derivative is characterized by a solubility at O°C in distilled water of at least 20 g/100 ml.

7. The use according to claim 6, wherein said cyclic oligosaccharide is alpha-, beta-, or gamma-cyclodextrin.

8. The use according to claim 6, wherein said cyclic oligosaccharide derivative is an anionic salt derivative of said oligosaccharide having substituents selected from the group consisting of sulfate, phosphate, carboxylate, and mixtures thereof associated with a physiologically acceptable cation.

9. The use according to claim 8, wherein said cyclic oligosaccharide is beta-cyclodextrin tetradecasulfate

10. The use according to claim 6, wherein said virus is a retrovirus.

11. Use of a carbohydrate blocking agent for the preparation of a medicament for inhibiting formation of syncytia between cells infected with a virus which is capable of interacting with said cells or virus, said carbohydrate blocking agent comprising a cyclic oligosaccharide derivative having up to about twelve sugar monomers, wherein said derivative is characterized by a solubility at O°C in distilled water of at least 20 g/100 ml.

12. The use according to claim 11, wherein said cyclic oligosaccharide is alpha-, beta-, or gamma-cyclodextrin.

13. The use according to claim 11, wherein said cyclic oligosaccharide derivative is an anionic salt derivative of said oligosaccharide having substituents selected from the group consisting of sulfate, phosphate, carboxylate, and mixtures thereof associated with a physiologically acceptable cation.

14. The use according to claim 13, wherein said cyclic oligosaccharide is beta-cyclodextrin tetradecasulfate.

15. The use according to claim 11, wherein said virus is a retrovirus.

16. The use according to claim 1, wherein said cyclic oligosaccharide derivative is used in combination with an antiviral agent.

17. The use according to claim 6, wherein said cyclic oligosaccharide derivative is used in combination with an antiviral agent.

18. The use according to claim 11, wherein said cyclic oligosaccharide derivative is used in combination with an antiviral agent.

19. The use according to claim 5, wherein said cyclic oligosaccharide derivative is used in combination with a latent cell growth modulating steroid or non-steroid organic compound wherein said steroid does not possess glucocorticoid activity.

20. The use according to claim 5, wherein said cyclic oligosaccharide derivative is used in combination with an antiviral agent and a latent cell growth modulating steroid or non-steroid organic compound wherein said steroid does not possess glucocorticoid activity.

## Patentansprüche

1. Verwendung eines Kohlenhydrat-Blockierungsmittels zur Herstellung eines Arzneimittels zur Inhibierung einer Virusinfektion von Zellen, welches in der Lage ist, mit den Zellen oder dem Virus zu interagieren, um die Bindung des Virus mit den Zellen zu stören, wobei das Kohlenhydrat-Blockierungsmittel ein cyclisches Oligosaccharid-Derivat mit bis zu etwa 1 2 Zuckermonomeren umfaßt, wobei das Derivat durch eine Löslichkeit bei 0 °C in destilliertem Wasser von mindestens 20 gm/100 ml gekennzeichnet ist.

2. Verwendung nach Anspruch 1, wobei das cyclische Oligosaccharid $\alpha$-, $\beta$- oder $\gamma$-Cyclodextrin ist.

3. Verwendung nach Anspruch 1, wobei das cyclische Oligosaccharid-Derivat ein anionisches Salz-Derivat des Oligosaccharids mit Substituenten ist, die aus der Gruppe von Sulfat, Phosphat, Carboxylat und Gemischen davon, assoziiert mit einem physiologisch verträglichen Kation, ausgewählt sind.

4. Verwendung nach Anspruch 3, wobei das cyclische Oligosaccharid $\beta$-Cyclodextrintetradecasulfat ist.

5. Verwendung nach Anspruch 1, wobei das Virus ein Retrovirus ist.

6. Verwendung eines Kohlenhydrat-Blockierungsmittels zur Herstellung eines Arzneimittels zur Inhibierung einer Virusübertragung von Zelle zu Zelle, welches in der Lage ist, mit den Zellen oder dem Virus zu interagieren, um die Bindung des Virus mit den Zellen zu stören, wobei das Kohlenhydrat-Blockierungsmittel ein cyclisches Oligosaccharid-Derivat mit bis zu etwa 12 Zuckermonomeren umfaßt, wobei das Derivat durch eine Löslichkeit bei 0 °C in destilliertem Wasser von mindestens 20 g/100 ml gekennzeichnet ist.

7. Verwendung nach Anspruch 6, wobei das cyclische Oligosaccharid $\alpha$-, $\beta$- oder $\gamma$-Cyclodextrin ist.

8. Verwendung nach Anspruch 6, wobei das cyclische Oligosaccharid-Derivat ein anionisches Salz-Derivat des Oligosaccharids mit Substituenten ist, die aus der Gruppe von Sulfat, Phosphat, Carboxylat und Gemischen davon, assoziiert mit einem physiologisch verträglichen Kation, ausgewählt sind.

16

**9.** Verwendung nach Anspruch 8, wobei das cyclische Oligosaccharid $\beta$-Cyclodextrintetradecasulfat ist.

**10.** Verwendung nach Anspruch 6, wobei das Virus ein Retrovirus ist.

**11.** Verwendung eines Kohlenhydrat-Blockierungsmittels zur Herstellung eines Arzneimittels zur Inhibierung der Bildung von Synzytien zwischen mit einem Virus infizierten Zellen, welches in der Lage ist, mit den Zellen oder dem Virus zu interagieren, wobei das Kohlenhydrat-Blockierungsmittel ein cyclisches Oligosaccharid-Derivat mit bis zu etwa 12 Zuckermonomeren umfaßt, wobei das Derivat durch eine Löslichkeit bei 0 °C in destilliertem Wasser von mindestens 20 g/100 ml gekennzeichnet ist.

**12.** Verwendung nach Anspruch 11, wobei das cyclisches Oligosaccharid $\alpha$-, $\beta$- oder $\gamma$-Cyclodextrin ist.

**13.** Verwendung nach Anspruch 11, wobei das cyclische Oligosaccharid-Derivat ein anionisches Salzderivat des Oligosaccharids mit Substituenten ist, die aus der Gruppe von Sulfat, Phosphat, Carboxylat und Gemischen davon, assoziiert mit einem physiologisch verträglichen Kation, ausgewählt sind.

**14.** Verwendung nach Anspruch 13, wobei das cyclische Oligosaccharid $\beta$-Cyclodextrintetradecasulfat ist.

**15.** Verwendung nach Anspruch 11, wobei das Virus ein Retrovirus ist.

**16.** Verwendung nach Anspruch 1, wobei das cyclische Oligosaccharid-Derivat in Verbindung mit einem Antivirusmittel verwendet wird.

**17.** Verwendung nach Anspruch 6, wobei das cyclische Oligosaccharid-Derivat in Verbindung mit einem Antivirusmittel verwendet wird.

**18.** Verwendung nach Anspruch 11, wobei das cyclische Oligosaccharid-Derivat in Verbindung mit einem Antivirusmittel verwendet wird.

**19.** Verwendung nach Anspruch 5, wobei das cyclische Oligosaccharid-Derivat in Verbindung mit einer, ein latentes Zellwachstum-modulierenden organischen Steroid- oder Nicht-Steroid-Verbindung verwendet wird, wobei das Steroid keine glucocorticoide Aktivität aufweist.

**20.** Verwendung nach Anspruch 5, wobei das cyclische Oligosaccharid-Derivat in Verbindung mit einem Antivirusmittel und einer, ein latentes Zellwachstum-modulierenden organischen Steroid- oder Nicht-Steroid-Verbindung verwendet wird, wobei das Steroid keine glucocorticoide Aktivität aufweist.

**Revendications**

**1.** Utilisation d'un agent bloquant à base d'hydrate de carbone pour la préparation d'un médicament pour inhiber l'infection de cellules par un virus, qui est capable d'interagir avec lesdites cellules ou virus en interférant au niveau de la liaison entre le virus et les cellules, ledit agent bloquant à base d'hydrate de carbone comprenant un dérivé oligosaccharide cyclique comportant jusqu'à environ 12 sucres monomères, dans laquelle ledit dérivé est caractérisé par une solubilité d'au moins 20 g/100 ml à 0 °C dans l'eau distillée.

**2.** L'utilisation selon la revendication 1, dans laquelle l'oligosaccharide cyclique est une alpha-, béta- ou gamma-cyclodextrine.

**3.** L'utilisation selon la revendication 1, dans laquelle ledit dérivé d'oligosaccharide cyclique est un dérivé salin anionique dudit oligosaccharide ayant des substituants choisis dans le groupe consistant en sulfate, phosphaste, carboxylate, et leurs mélanges associés avec un cation physiologiquement acceptable.

**4.** L'utilisation selon la revendication 3, dans laquelle ledit oligosaccharide cyclique est du tétradecasulfate de béta cyclodextrine.

**5.** L'utilisation selon la revendication 1, dans laquelle ledit virus est un rétrovirus.

17

6. L'utilisation d'un agent bloquant à base d'hydrate de carbone pour la préparation d'un médicament pour l'inhibition de la transmission cellule à cellule d'un virus, qui est capable d'interagir avec lesdites cellules ou virus en interférant au niveau de la liaison entre le virus et les cellules, ledit agent bloquant à base d'hydrate de carbone comprenant un dérivé d'oligosaccharide cyclique ayant jusqu'à environ 12 sucres monomères, dans laquelle ledit dérivé est caractérisé par une solubilité d'au moins 20 g/100 ml à 0°C dans l'eau distillée .

7. L'utilisation selon la revendication 6, dans laquelle ledit oligosaccharide cyclique est une alpha-, béta-, ou gamma-cyclodextrine.

8. L'utilisation selon la revendication 6, dans laquelle ledit dérivé d'oligosaccharide cyclique est un dérivé salin anionique dudit oligosaccharide ayant des substituants choisis dans le groupe consistant en sulfate, phosphaste, carboxylate, et leurs mélanges associés avec un cation physiologiquement acceptable.

9. L'utilisation selon la revendication 8, dans laquelle ledit oligosaccharide cyclique est du tétradécasulfate de béta cyclodextrine.

10. L'utilisation selon la revendication 6, dans laquelle ledit virus est un retrovirus.

11. L'utilisation d'un agent bloquant à base d'hydrate de carbone pour la préparation d'un médicament pour inhiber la formation de syncytie entre des cellules infectées par un virus, qui est capable d'interagir avec lesdites cellules ou virus, ledit agent bloquant à base d'hydrate de carbone comprenant un dérivé d'oligosaccharide cyclique ayant jusqu'à environ 12 sucres monomères, dans laquelle ledit dérivé est caractérisé par une solubilité d'au moins 20 g/100 ml à 0°C dans l'eau distillée .

12. L'utilisation selon la revendication 11, dans laquelle ledit oligosaccharide cyclique est une alpha-, béta- ou gamma-cyclodextrine.

13. L'utilisation selon la revendication 11, dans laquelle ledit dérivé d'oligosaccharide cyclique est un dérivé salin anionique dudit oligosaccharide ayant des substituants choisis dans le groupe consistant en sulfate, phosphaste, carboxylate, et leurs mélanges associés avec un cation physiologiquement acceptable.

14. L'utilisation selon la revendication 13, dans laquelle ledit oligosaccharide cyclique est le tétradécasulfate de beta cyclodextrine.

15. Utilisation selon la revendication 11, dans laquelle ledit virus est un rétrovirus.

16. L'utilisation selon la revendication 1, dans laquelle ledit dérivé d'oligosaccharide cyclique est utilisé en combinaison avec un agent antiviral.

17. L'utilisation selon la revendication 6, dans laquelle ledit dérivé d'oligosaccharide cyclique est utilisé en combinaison avec un agent antiviral.

18. L'utilisation selon la revendication 11, dans laquelle ledit dérivé d'oligosaccharide cyclique est utilisé en combinaison avec un agent antiviral.

19. L'utilisation selon la revendication 5, dans laquelle ledit dérivé d'oligosaccharide cyclique est utilisé en combinaison avec un composé organique stéroïdien ou non-stéroïdien modulant la croissance cellulaire latente, ledit composé stéroïdien ne possédant pas d'activité glucocorticoïde.

20. L'utilisation selon la revendication 5, dans laquelle ledit dérivé d'oligosaccharide cyclique est utilisé en combinaison avec un agent antiviral et un composé organique stéroïdien ou non-stéroïdien modulant la croissance cellulaire latente, ledit composé stéroïdien ne possédant pas d'activité glucocorticoïde.

## FIG. 1A

## FIG. 1B

# FIG. 2

# FIG. 3A

# FIG. 3B

# FIG. 4

FIG. 5

COMPARISON OF SYNCYTIA INHIBITION

HIV − 1

FIG. 6a

# FIG.6b